# EUROPEAN PATENT APPLICATION

(11) **EP 2 371 324 A1**
(43) Date of publication of application: **05.10.2011**
(21) Application number: 11151613.4
(22) Date of filing: 21.01.2011
(51) Int. Cl.: A61F 7/00, A61F 7/02

(54) **Portable heating pad with improved structure**

(30) Priority: 31.03.2010 IT MI20100549
(71) Applicant: Tenacta Group S.p.A., 24052 Azzano S. Paolo (BG) (IT)
(72) Inventor: MORGANDI, Arturo, I-24100, Bergamo (IT); MILANI, Giancarlo, I-24030, Mozzo (BG) (IT)
(74) Representative: Pistolesi, Roberto

(57) **Abstract**

A heating pad with electric heating means and a phase change material for maintaining the temperature comprises at least one heating element (11) which comprises in turn a rigid and flat outer casing (12, 13) which contains a heating module (14). Each module comprises a central supporting base-piece (24) provided with electric heating resistors (25) and two layers (27, 28) which are arranged on opposite faces of the base-piece and are made of a phase change material with a transition temperature which corresponds to the desired internal operating temperature of the heating pad.

## Description

### DESCRIPTION

The present invention relates to a heating pad with electric heating and heat storage means, which makes use of the latent heat due to the phase change of a suitable material.

In the sector of heating pads (generally used for localized heating of parts of the body) apparatus which contain a heat storage mass and an electric circuit for heating this mass have been proposed. Once the storage mass has reached the desired temperature by means of the electric circuit, the apparatus may thus be disconnected from the electric power supply and used until it cools.

In order to attempt to obtain a temperature which is relatively constant during use, heating pads have been proposed where the heat storage mass is formed at least partially with a suitable material which undergoes a change in state at the desired temperature of use. In this way, by heating the heating pad mentioned to just above the temperature where a change in state occurs, it is possible to obtain a release of heat at a constant temperature for the entire period of time required for the solidification state change.

Usually the phase change material is made in the form a thin pad which is inserted inside a flexible casing. When, however, the phase change material is converted from the solid state to the liquid or paste-like state it undergoes deformation which, after cooling, generally results in the heating pad assuming a deformed, and often twisted, rigid condition. As well as having an appearance which is unacceptable from an aesthetic point of view, which gives the user the impression that the product is defective and/or of poor quality, the deformation may also result in there no longer being optimum contact between the phase change material and the electric heating circuit, with a reduction in the efficiency of the apparatus during subsequent use. Furthermore, problems during heating also exist in the case of a rigid structure needed to ensure a better containing action.

The use of a totally rigid containing structure also results in difficulty of use, especially if it is required to have a heating pad with a relatively large surface area, which must be placed on a relatively broad zone of the body, such as the stomach, back or shoulders. The problem is increased by the need to have a containing structure which is strong and prevents excessive mechanical stressing of the electric heating part.

Apparatus of the abovementioned type have been described, for example, in EP1.894548, WO96/26694 WO00/24348, US2005/0021115 and GB2160965.

The main object of the present invention is to provide a heating pad which maintains an optimum shape of the phase change material, is strong and offers suitable protection of the internal components. A further object is to provide a heating pad which is easy and comfortable to use.

In view of these objects the proposed idea according to the invention is to provide a heating pad according to Claim 1.

In order to illustrate more clearly the innovative principles of the present invention and its advantages compared to the prior art, an example of embodiment applying these principles will be described below, with the aid of the accompanying drawings. In the drawings:
- Figure 1 shows an exploded perspective view of a heating pad provided according to the present invention;
- Figure 2 shows a partial, schematic, cross-sectional view of detail of the heating pad according to Figure 1;
- Figure 3 shows an exploded, schematic, perspective view of a heating detail of the heating pad according to Figure 1;
- Figure 4 shows a perspective view of the heating pad according to Figure 1 assembled and inserted inside a protective bag.

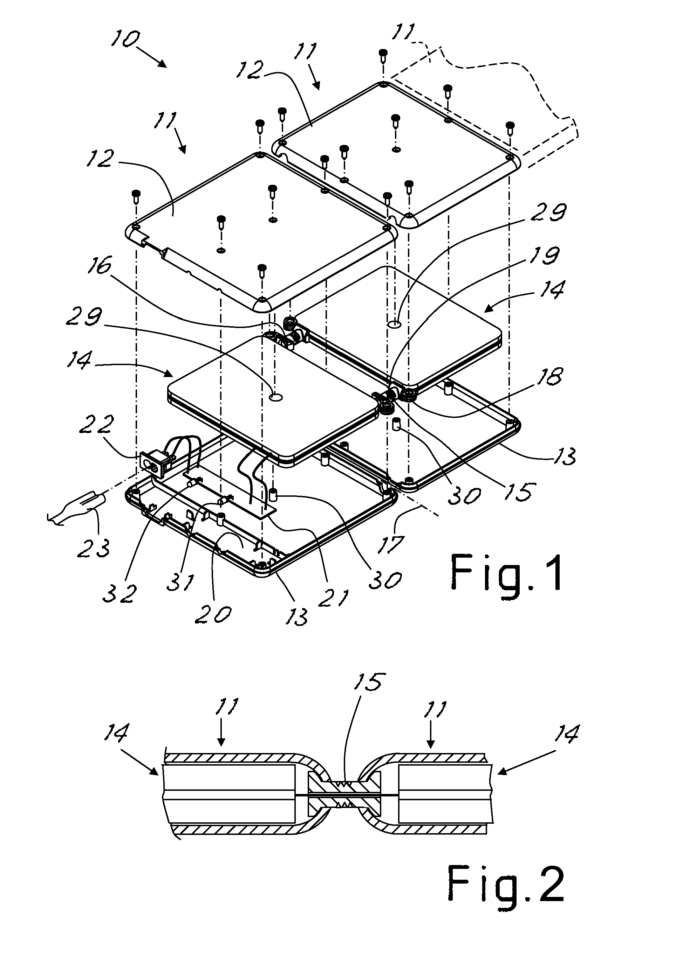

With reference to the figures, Figure 1 shows a heating pad according to the invention, denoted generally by 10.

The heating pad 10 comprises at least one heating element 11 which comprises two matching shells 11, 12 (advantageously made of rigid plastic) which are joined together to form a flat rectangular casing (for example with dimensions 12x12 cm), advantageously with rounded edges, which contains a respective heat module 14. The figures show two hinged elements, but, from the description which follows, it will be easy for the person skilled in the art to imagine a version with just one element.

The module 14 may advantageously have a central hole 29 for centring on pins 30 projecting from the bottom of the containing shells 12, 13. The pins may also help keep the module centred in the thickness between the shells. The height of the internal space of the shells joined together is advantageously such as to house the heat modules with a minimum play at least in the direction perpendicular to their extension.

Advantageously, at least one pair of heating elements 11 which are hinged in sequence in their plane of extension so as to be articulated together and inclinable are used.

The hinging together of the two elements 11 is advantageously achieved along a side edge by means of a pair of (flexible or articulated) joints 15, 16 which are connected so as to form a bridge between facing sides of the two elements 11. Advantageously, the two joints are arranged close to two opposite edges of one side of the element 11.

The two joints 15, 16 allow advantageously the relative inclination of the two elements 11 about their facing sides (namely about an axis 17 transverse to the extension of the pair of interconnected elements), avoiding at the same time an uncontrolled relative rotation about an axis parallel to this extension, so that the facing side edges of the two elements 11 remain substantially parallel to each other.

In the embodiment shown, the joints are advantageously made of flexible plastic and have a generally elongate farm with their axis directed perpendicular to the interconnected sides. For improved flexibility they also have suitable lateral incisions.

For a safe connection, each joint comprises advantageously two rings 18, 19, each one of which is close to one end of the joint situated inside the respective casing of the element 11, so as to be engaged with a suitable pin which forms the seat for a screw for closing the shells.

The first or single heating element 11 comprises internally a compartment 20 which houses an electronic base-piece with a control system 21 connected to the heating modules. The control system 21 receives the electric power supply via a socket 22 which is flush-mounted on an edge of the element 11 and which is intended to be connected to an electricity source by means of an electrical power supply cable provided with a mating connector 23. The control system 21 may advantageously comprise a microprocessor unit which is suitably programmed to control operation of the heating pad, as will become clear below.

As shown in Figure 2, when several modules are present, the electrical connections between the heating modules pass advantageously axially through the joints 15, 16. Advantageously an outgoing conductor passes through one of the joints, while the return conductor passes through the other joint. The joints may be designed so that they can be opened or the conductors may be separated for axial introduction into the joints. Alternatively, the joint may also be moulded directly onto the respective conductor.

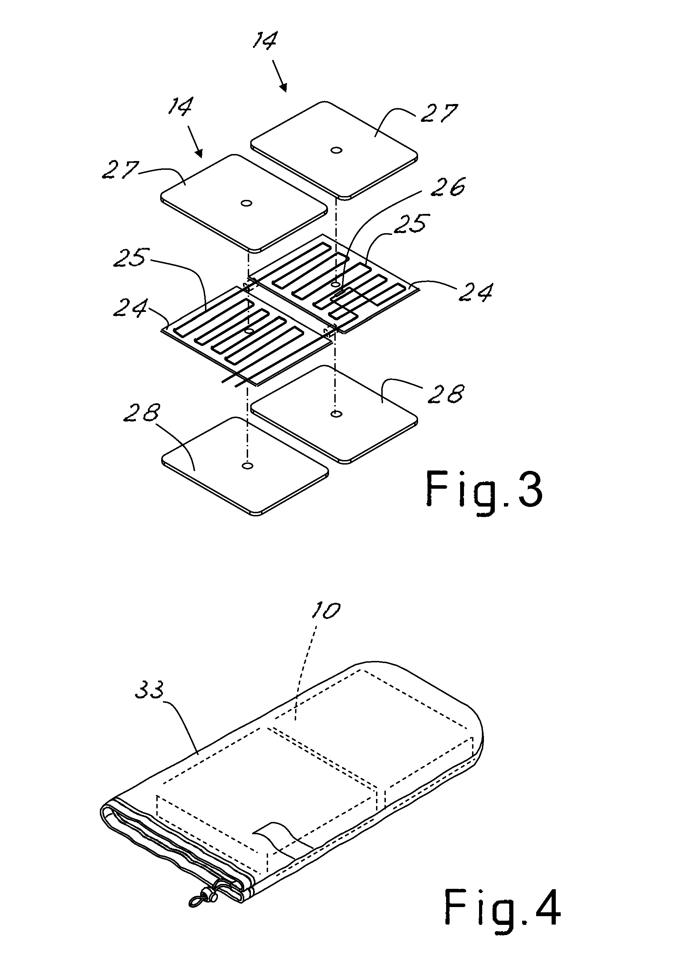

Figure 3 shows the structure of the heating modules 14 which has been found to be particularly advantageous both in the case of a single element and in the case of several hinged elements. As can be seen in this figure, each module 14 comprises a support 24 (preferably in the form of a flat central supporting base-piece) which is made of electrically insulating and preferably rigid material (for example a suitable plastic which is able to withstand the heat produced by heating of the heating pad) and which contains the electric heating resistor 25 arranged in alternating loops so as to distribute the heat uniformly over the entire surface of the base-piece. In the case of several hinged elements, the resistors of the various modules may be advantageously connected in series, as can be clearly seen in Figure 3. As described above, the electrical connections between one base-piece and the other base-piece pass through the joints 15, 16. In the case of temperature sensors separated by the electric resistors, the connections between these sensors and the control unit may also pass through the articulated joints.

Advantageously, at least one base-piece (or the single base-piece in the case of a heating pad composed of a single element 11) may be provided with a safety thermostat 26 which deactivates the resistors in the event of a predefined limit temperature being exceeded.

The opposite faces of each base-piece are provided with two layers 27, 28 of a known phase change material (PCM) which is solid at room temperature and has a suitable phase transition temperature (melting temperature) which corresponds to the desired internal operating temperature of the heating pad.

Advantageously, the phase change temperature of the material is chosen so as to be between 55°C and 60°C and, in particular, about 58°C.

The volume of the layers will be chosen so as to produce a desired duration for release of latent heat during use of the heating pad. The PCM will be advantageously of the known type formed by a paraffin inserted in a polymer matrix or grid. This type of material has the advantage that, once its phase change temperature has been exceeded, it softens from the solid state, but the polymer structure prevents complete liquefaction of the entire layer.

During use of the heating pad, it is firstly connected to the electricity source in order to heat suitably the heat modules. The control system 21 (which is of the known type and therefore not further described) will power the modules so as to reach and maintain, where necessary, the programmed phase change temperature. For this purpose the control system may also comprise one of more temperature sensors, known per se, for detecting the temperature of the modules. The presence of the power supply may be signalled by a first indicator lamp 31, for example an LED.

The layers of PCM remain advantageously confined within the space between the respective face of the central base-piece and the facing inner side of the rigid casing. The cohesion of the structure and the thermal connection are always maintained.

At the end of heating (if necessary signalled by the switching on or off of a second indicator lamp 32, for example an LED) it is possible to remove the plug 23 from the socket 22 and use the heating pad until it cools, with the temperature which is kept substantially constant until the end of the solidification state change.

Advantageously, when the heating pad is used, it may also be inserted inside a suitable bag 33 made of flexible material, such as fabric or the like, as shown in Figure 4.

At this point it is clear how the predefined objects have been achieved. Owing to the structure according to the invention, it is possible to ensure cohesion between the layers of PCM and their correct connection to the walls of the casing and to the heating resistors. With the structure according to the invention, the heat exchange efficiency has been found to be surprisingly improved both during heating and during use. Moreover, in the case where a heating pad which is suitable for large areas is required, by providing several modules hinged together, the hinged arrangement of the elements 11 (which are advantageously square in plan view) allows the heating pad to be easily adapted to the body. Relatively low manufacturing costs are also possible owing to the simple structure.

Obviously, the above description of an embodiment applying the innovative principles of the present invention is provided by way of example of these innovative principles and must therefore not be regarded as limiting the scope of the rights claimed herein. The figures show only two interconnected elements 11, but it is clear that, by applying the principles of the present invention, it is possible to provide heating pads with any desired number of elements arranged in sequence (as shown schematically in broken lines in Figure 1). For this purpose, the intermediate elements will have the connection with the articulated joints repeated on their opposite sides, as can be easily imagined by the person skilled in the art on the basis of the description provided above. It is thus possible to form a belt for the stomach or a "scarf" for the shoulders and the neck. Owing to the modular structure according to the invention, heating pads of different length with a different number of elements connected in series may also be produced at a minimum cost. If desired or preferred for particular requirements, the resistors of the heating modules may also be connected in parallel, instead of in series, or a combination of the two.

## Claims

1. Heating pad with electric heating and heat storage means and a phase change material for maintaining the temperature, **characterized in that** it comprises at least two heating elements which are connected together in sequence in a hingeable manner, each heating elements (11) comprising in turn a rigid and flat outer casing (12, 13) which contains a heating module (14) comprising a support piece (24) provided with electric heating resistors (25) and two layers (27, 28) which are arranged on the opposite faces of the support piece and are made of phase change heat storage material with a transition temperature which corresponds to the desired internal operating temperature of the heating pad, conductors passing between the rigid outer casing for electrical connection of the electric heating resistors (25) for connecting the heating pad to a electricity source for its electrical heating.

2. Heating pad according to Claim 1, **characterized in that** the hinging system comprises two joints (15, 16) connected so as to form a bridge between two facing sides of two interconnected heating elements (11).

3. Heating pad according to Claim 2, **characterized in that** the joints (15, 16) are made of flexible plastic and have a generally elongate form with their axis directed perpendicular to the facing sides of the interconnected elements.

4. Heating pad according to Claim 1, **characterized in that** at least one heating module (14) comprises a safety thermostat (26) in series with the electrical heating resistors (25).

5. Heating pad according to Claim 2, **characterized in that** the conductors for electrically connecting the electric heating resistors (25) pass through the joints (15,16).

6. Heating pad according to Claim 1, **characterized in that** one of the heating elements comprises a socket (22) for connecting the heating pad to an electricity source.

7. Heating pad according to Claim 1, **characterized in that** a heating element comprises a compartment (20) containing an electronic control system (21) connected to the heating modules.

8. Heating pad according to Claim 1, **characterized in that** the phase change material of the layers (27, 28) comprises a paraffin inserted in a polymer matrix or grid.

9. Heating pad according to Claim 1, **characterized in that** the phase change temperature of the material of the layers (27, 28) is between 55°C and 60°C and, in particular, about 58°C.

10. Heating pad according to Claim 1, **characterized in that** it comprises an external bag (33) made of flexible material, such as fabric or the like.
